# EUROPEAN PATENT APPLICATION

(11) **EP 4 603 104 A1**
(43) Date of publication of application: **20.08.2025**
(21) Application number: 23884410.4
(22) Date of filing: 29.08.2023
(51) Int. Cl.: A61K 38/09, A61P 5/24, C07K 16/26, A61K 38/24, C07K 14/59, A61P 15/08

(54) **USE OF GNRH ANTAGONIST IN REGULATION AND CONTROL OF ESTRUS OF MAMMAL**

(30) Priority: 04.11.2022 CN 202211376492
(71) Applicant: Beijing Vjtbio Co., Ltd., Beijing 100085 (CN)
(72) Inventor: LUO, Haoshu, Beijing 100085 (CN); ZHANG, Jiawei, Beijing 100085 (CN); SHI, Lei, Beijing 100085 (CN); HAN, Guo, Beijing 100085 (CN)
(74) Representative: reuteler & cie SA
(86) International application number: PCT/CN2023/115357
(87) International publication number: WO 2024/093473

(57) **Abstract**

The present invention relates to the field of mammalian reproduction. Disclosed is use of a GnRH antagonist in the regulation and control of estrus of a mammal. The GnRH antagonist can inhibit oestrus for a longer time with less frequent use, and can be used for estrus synchronization and batch reproduction of livestock husbandry animals or regulation and control of estrus of pets.

## Description

### CROSS-REFERENCE TO RELATED APPLICATIONS

The application claims priority to the Chinese Application No. 202211376492.5, filed on November 4, 2022, the content of which is specifically and entirely incorporated herein by reference.

### FIELD

The present disclosure relates to the field of mammalian reproduction, in particular to an use of a GnRH antagonist in the regulation and control of oestrus of a mammal, a pharmaceutical composition, an use of GnRH antagonist and gonadotrophin for regulation and control of oestrus synchronization of a mammal, a method for inhibiting oestrus of a mammal, and a method for regulation and control of oestrus synchronization of mammals.

### BACKGROUND

GnRH antagonists are GnRH analogs, in which GnRH is a decapeptide secreted by the hypothalamus, and can bind with GnRH receptors, activate downstream signaling pathways, and promote the secretion of gonadotrophin in mammals, thus it is a key regulatory factor in the hypothalamus-pituitary-gonad axis. GnRH antagonists are analogs of GnRH that have been modified and replaced with amino acids, which can competitively bind to GnRH receptors and block downstream signaling pathways, thereby inhibiting the secretion of gonadotrophin in animal body. Based on the physiological function of GnRH, GnRH antagonists, including Abarelix, Cetrorelix and Degarelix, among others, are currently used clinically primarily in the treatment of prostate cancer in males, infertility and endometriosis in females.

With the improvement of living standards, the consumption of livestock products has been continuously upgraded, and the demand for high-quality and safe livestock products has been increasing. At present, oestrus synchronization and other means are used in the breeding process of livestock husbandry animals to improve the breeding efficiency, thereby increasing the farming benefits and product supply. In the breeding process of cattle and sheep, vaginal suppositories containing progestin are mainly used to facilitate oestrus synchronization of female animals, while in the breeding process of swines, altrenogest is used for continuous feeding for 18 days to synchronize oestrus of gilts. Livestock husbandry animals treated with vaginal suppositories or altrenogest can achieve the synchronous oestrus, and then carry out the batch production to improve breeding efficiency and economic benefits. Both vaginal suppositories and altrenogest are artificially synthesized progestin, with the risk of polluting the environment and affecting physical health. The vaginal suppositories are applied on the cattle and sheep to regulate and control oestrus, but long-term use of vaginal suppositories may cause the falling of suppositories, resulting in premature oestrus, or may cause vaginal adhesions and infections that affect health conditions of animals; and altrenogest needs to be fed continuously for 18 days, the cumbersome operation increases labor costs, and multiple feedings increase the risk of disease transmission.

There are many measures to inhibit oestrus in pets, which can be divided into surgical methods and non-surgical methods. In the practical applications at present, surgical methods are the most widely used methods. However, sterilization operation is prone to bring some problems. Firstly, sterilization operation is irreversible for pets, which means pets cannot restore their fertility after sterilization operation. Secondly, sterilization operation will also bring forth some risks to the health of pets, which may threaten the life of pets. Postoperative complications such as bleeding and infection will also affect the health of pets. The most important thing is that sterilization brings a high incidence of some diseases to pets, such as cruciate ligament rupture, hemangioendothelioma, lymphoma, bladder cancer, and over-obesity. In addition, pets after sterilization operation are prone to suffer from urinary incontinence, which seriously affects the health and life quality of pets. At the same time, there are a few pets that use oestrus suppression drugs, such as Suprelorin manufactured by the Virbac Company in France, which can be used to inhibit oestrus in male dogs. However, it may have side effects in the early stages of use in male dogs, such as aggressiveness, strong sexual desire, and urine marking behavior; moreover, the side effects of said drugs when used in female dogs are excessively fierce, such as inducing oestrus phenomenon in female animals, and causing uterine diseases, ovarian cysts, pseudopregnancy, urinary incontinence, cystitis, weight gain, fur and behavior changes, etc., making it impossible to use the drugs clinically in female dogs.

### SUMMARY

The present disclosure aims to overcome the defects in the prior art, and provides an use of a GnRH antagonist in regulation and control of oestrus of a mammal, the GnRH antagonist can inhibit oestrus for a longer time with less frequent use, and can be used for oestrus synchronization and batch reproduction of livestock husbandry animals or regulation and control of oestrus of pets.

In order to achieve the above-mentioned objects, the first aspect of the present disclosure provides a use of a GnRH antagonist in regulation and control of oestrus of a mammal.

Preferably, the use is to inhibit oestrus or regulate and control oestrus synchronization.

Preferably, the GnRH antagonist is at least one selected from the group consisting of Cetrorelix, Abarelix and Degarelix; more preferably is Degarelix.

The second aspect of the present disclosure provides a pharmaceutical composition comprising a GnRH antagonist and a gonadotrophin.

Preferably, the gonadotrophin is at least one selected from the group consisting of follicle stimulating hormone (FSH) and analogs thereof, pregnant mare serum gonadotropin (PMSG) and analogs thereof, and human chorionic gonadotropin (HCG) and analogs thereof.

The third aspect of the present disclosure provides a use of GnRH antagonist and gonadotrophin for regulation and control of oestrus synchronization of a mammal.

The fourth aspect of the present disclosure provides a method for inhibiting oestrus of a mammal, wherein the method comprises: administering an effective amount of a GnRH antagonist to the non-estrous mammal to inhibit oestrus of the mammal.

The fifth aspect of the present disclosure provides a method for regulation and control of oestrus synchronization of mammals, wherein the method comprises: administering an effective amount of a GnRH antagonist to the non-estrous mammals to inhibit oestrus of mammals, and then administering an effective amount of gonadotrophin to achieve oestrus synchronization.

Compared to the traditional drugs for inhibiting oestrus, the use of a GnRH antagonist in the present disclosure to inhibit mammal oestrus can considerably reduce the frequency of administration and allows a long term inhibition effect to be maintained through one injection, in particular, Degarelix can produce a desirable inhibitive efficacy at the same dosage, and the inhibition effect is eliminable without causing permanent non-estrous, thus it is particularly suitable for oestrus inhibition of pets.

The pharmaceutical composition of the present disclosure is particularly suitable for use in reproduction and breeding of livestock husbandry animals, since an use of a GnRH antagonist can achieve long term inhibition of oestrus in the female animals, followed by the administration of gonadotrophin to induce estrus of the animals, thereby fulfilling the purpose of oestrus synchronization.

### BRIEF DESCRIPTION OF THE DRAWINGS

FIG. 1 shows the oestrus synchronization graph of the female mice injected with 1.0mg/kg Degarelix in Example 2 of the present disclosure;
FIG. 2 illustrates the oestrus synchronization graphs of gilts treated with altrenogest (a) and Degarelix (b) in Example 3 of the present disclosure, respectively;
FIG. 3 shows graphs indicating the curve change in testosterone concentration (a) and scrotum circumference (b) in male dogs treated with Degarelix in Example 4 of the present disclosure, respectively, wherein, ▲ represents a control group, and ■ represents a Degarelix treatment group.

### DETAILED DESCRIPTION

The terminals and any value of the ranges disclosed herein are not limited to the precise ranges or values, such ranges or values shall be comprehended as comprising the values adjacent to the ranges or values. As for numerical ranges, the endpoint values of the various ranges, the endpoint values and the individual point value of the various ranges, and the individual point values may be combined with one another to produce one or more new numerical ranges, which should be deemed have been specifically disclosed herein.

Hormonal biological products, whose chemical composition may not be constant or whose quality specifications cannot yet be determined by physicochemical methods, are often tested for potency by comparing biological experimental methods with standards. Through biological identification, the minimum potency unit with a certain biological efficacy is called unit (U); the standard unit stipulated through international consultation is called international unit (IU). The conversion of units and weights of different drugs is not the same. Therefore, in the present disclosure, the units used are based on the measurement units provided for the goods purchased, wherein the measurement units of GnRH antagonists use "mg/kg" or "µg/mg", the measurement units of each of FSH, PMSG and HCG use "units".

The first aspect of the present disclosure provides a use of a GnRH antagonist in regulation and control of oestrus of a mammal.

Preferably, the use is to inhibit oestrus or regulate and control oestrus synchronization.

The species of the mammal is not particularly limited, and in general, the mammal may be a cat, a dog, a mouse, a rabbit, a horse, a cattle, a sheep or a pig. Wherein, for livestock husbandry animals such as horse, cattle, sheep or pig, the GnRH antagonist can be used to regulate and control oestrus synchronization as required, so as to achieve batch reproduction, the subject to be administered is usually female animals. Whilst inhibiting oestrus, there is no requirement on the gender of the mammal, both of the male and female animals can be administered, although the action mechanism may be different.

Preferably, the GnRH antagonist is at least one selected from the group consisting of Cetrorelix, Abarelix and Degarelix; more preferably is Degarelix.

Those skilled in the art can adjust dosage of GnRH antagonist according to the particular mammal species and the purposes of administration. Generally speaking, dosage of the GnRH antagonist is 1-5,000 µg per kg of animal body weight. For example, for rodents such as mice and rabbits, dosage of the GnRH antagonist is 1,000-2,000 µg/kg body weight; for medium and large livestock husbandry animals such as pigs, cattles, sheep, and horses, dosage of the GnRH antagonist is 1-100 µg/kg body weight; for pets such as dogs and cats, dosage of the GnRH antagonist is 1,000-5,000 µg/kg body weight.

The present disclosure also relates to use of the GnRH antagonist in preparation of a product for regulation and control of oestrus of a mammal. The product may be in the forms such as liquid, solid, capsule, injection agent, which acts as an oestrus inhibitor.

The GnRH antagonist may act in conjunction with other substances to regulate and control oestrus of a mammal, including but not limited to the use of a GnRH antagonist in conjunction with gonadotrophin, steroidal sex hormones, prostaglandins and analogs thereof.

The second aspect of the present disclosure provides a pharmaceutical composition comprising a GnRH antagonist and a gonadotrophin.

For description of the GnRH antagonist, please refer to the first aspect.

Preferably, the gonadotrophin is at least one selected from the group consisting of follicle stimulating hormone (FSH) and analogs thereof, pregnant mare serum gonadotropin (PMSG) and analogs thereof, and human chorionic gonadotropin (HCG) and analogs thereof.

Those skilled in the art can adjust dosage of gonadotrophin according to the specific mammalian species. Generally speaking, dosage of gonadotrophin is within the range of 5-6,000 units. Specifically, for example, for murine, dosage of follicle stimulating hormone (FSH) and analogs thereof is within the range of 5-10 units, dosage of PMSG and analogs thereof is within the range of 5-10 units, and dosage of HCG and analogs thereof is within the range of 5-10 units; for sowpigs, dosage of follicle stimulating hormone (FSH) and analogs thereof is within the range of 1,000-2,000 units, dosage of PMSG and analogs thereof is within the range of 500-1,500 units, and dosage of HCG and analogs thereof is within the range of 500-1,000 units.

GnRH antagonist and gonadotrophin in the pharmaceutical composition may be stored independently and act respectively to implement oestrus synchronization for different phases, thereby achieving the oestrus synchronization effect.

The third aspect of the present disclosure provides a use of GnRH antagonist and gonadotrophin for regulation and control of oestrus synchronization of a mammal.

For description of GnRH antagonist, please refer to the first aspect; as regards the description of gonadotrophin, please refer to the second aspect; with respect to the specific administration methods, please refer to the fifth aspect.

The fourth aspect of the present disclosure provides a method for inhibiting oestrus of a mammal, wherein the method comprises: administering an effective amount of a GnRH antagonist to the non-estrous mammal to inhibit oestrus of the mammal.

In the present disclosure, an effective amount of a GnRH antagonist refers to the amount that can appropriately and effectively inhibit oestrus or control the oestrus inhibition time within a certain range under the circumstance. It should be understood that effective amount of the GnRH antagonist may vary significantly depending on the species of the mammal and the specific needs, as for the specific content, please refer to the first aspect.

The administration method is not particularly limited and may be any conventional administration method in the art, such as at least one selected from the group consisting of intravenous injection, intramuscular injection, subcutaneous injection, subcutaneous implantation and vaginal suppository.

It should be understood that those skilled in the art can select the frequency of administration according to the type, efficacy and dosage of the GnRH antagonist as well as species of animals, for example, the GnRH antagonist can be administered once at an interval to produce the effect of selectively adjusting the time of inhibiting oestrus. Generally speaking, inhibiting oestrus of mammals is often suitable for pets, such as cats or dogs. The key requirement of pets is to inhibit oestrus, instead of the oestrus synchronization, and the injection dosage may allow the inhibition time to be more than 3 months.

The fifth aspect of the present disclosure provides a method for regulation and control of oestrus synchronization of mammals, wherein the method comprises: administering an effective amount of a GnRH antagonist to the non-estrous mammals to inhibit oestrus of mammals, and then administering an effective amount of gonadotrophin to achieve the oestrus synchronization.

For the explanation of the effective amount, please refer to the fourth aspect. For the gonadotrophin, its effective amount refers to the amount that can appropriately and effectively control estrus synchronization in this situation. For details, please refer to the second aspect.

As for the method of administering the GnRH antagonist, please refer to the fourth aspect. In order to achieve oestrus synchronization, the dosage of the GnRH antagonist can be adjusted so that oestrus inhibition time of the mammal is within a certain range, preferably, 7-35 days after administration of the GnRH antagonist (the animal is still in oestrus suppression state), 5-6,000 units of gonadotrophin are administered to achieve oestrus synchronization.

The method for administering gonadotropin may be a conventional method in the art. Preferably, the method for administering gonadotropin is as follows: administering an effective amount of gonadotrophin (at least one selected from the group consisting of FSH and analogs thereof, PMSG and analogs thereof, and HCG and analogs thereof, more preferably PMSG or HCG) to a mammal all at once; or initially administering an effective amount of at least one selected from the group consisting of FSH and analogs thereof and PMSG and analogs thereof (more preferably PMSG) to the mammal, and then administering HCG or analogs thereof after 24-96h. Those skilled in the art can select a suitable administration method according to the species of animal.

The mode of administering gonadotrophin is not particularly limited in the present disclosure, it may be a conventional administration mode in the art, for example, it may be at least one selected from the group consisting of intramuscular injection, subcutaneous injection, oral administration, subcutaneous implantation, and vaginal suppository.

The mode of administering the gonadotrophin and the mode of administering the GnRH antagonist may be the same or different.

Preferably, the mammal is a horse, a cattle, a sheep or a pig. It shall be understood that the use of oestrus synchronization is often applicable to female animals, such as a sowpig, a cow or a ewe.

In a preferred embodiment of the present disclosure, a method for regulation and control of oestrus synchronization of sowpigs is provided, the method comprises: administering 10-20µg/kg of Degarelix to gilts, and administering 500-1,500 units of PMSG or an analog thereof after 18-35 days to achieve oestrus synchronization of gilts.

In a preferred embodiment of the present disclosure, a method of controlling oestrus synchronization of female mice is provided, the method comprises: administering 0.5-2mg/kg of Degarelix to the female mice, administering 5-10 units of PMSG or an analog thereof after 7-35 days, and then administering 5-10 units of HCG or an analog thereof after 46-48 hours, in order to achieve oestrus synchronization of the female mice.

Those skilled in the art can refer to the above methods to carry out oestrus synchronization treatment on other mammals, and the content will not be described in detail herein.

In the present disclosure, GnRH antagonist and gonadotrophin are commercially available, and may be administered according to the corresponding instructions for use of the particular products.

The present disclosure will be described in details below with reference to examples.
Abarelix, Cetrorelix and Degarelix were purchased from Acmec;
PMSG was purchased from Ningbo Sansheng Biological Technology Co., Ltd.;
HCG was purchased from Ningbo Sansheng Biological Technology Co., Ltd.

### Example 1

This example served to illustrate screening of GnRH antagonists to inhibit oestrus of female mice.

The effects of three different GnRH antagonists (Abarelix, Cetrorelix and Degarelix) on inhibiting oestrus were compared.

Twenty-four 6-week-old female mice were selected and divided into 4 groups, with 6 mice in each group. The female mice in the negative control group were subcutaneously injected with normal saline, the female mice in the Abarelix group were subcutaneously injected with a dosage of 2 mg/kg/day for 8 consecutive days, the female mice in the Cetrorelix group were subcutaneously injected with a dosage of 2 mg/kg/day for 8 consecutive days, and the female mice in the Degarelix group were subcutaneously injected once with a dose of 2 mg/kg. After administration, vaginal smear experiments were performed on female mice every day to detect the stage of the oestrus cycle of female mice in each group. The results of vaginal smears showed that each of the three GnRH antagonists could inhibit oestrus of female mice, and oestrus could be restored after a period of inhibition.

As shown in Table 1, continuous injection of Abarelix for 8 days can effectively suppress oestrus of female mice for more than 11 days; continuous injection of Cetrorelix for 8 days can effectively inhibit oestrus of female mice for more than 12 days; and only once injection of Degarelix can inhibit oestrus of female mice for a long time, which is more than 28 days.

**Table 1**

| Groups | Treatment | Time of inhibiting oestrus (days) |
|---|---|---|
| Negative control group | Injection of normal saline | 4.67 ± 0.49^{a} |
| Abarelix experimental group | 2mg/kg/day, continuous subcutaneous injection for 8 days | 11.83 ± 0.72^{b} |
| Cetrorelix experimental group | 2mg/kg/ day, continuous subcutaneous injection for 8 days | 13.50 ± 1.31^{c} |
| Degarelix experimental group | 2mg/kg, only subcutaneous injection once | 34.50 ± 5.25^{d} |

| | | |
|---|---|---|
| Note: the different letters of the shoulder markers indicate significant differences (P *<* 0.05). | | |

### Example 2

This example served to illustrate use of Degarelix for inhibiting oestrus of female mice and achieving oestrus synchronization.

The duration of oestrus inhibition in female mice after administration with different dosages of Degarelix was tested, and the oestrus-inhibited female mice were subjected to the treatment of oestrus synchronization.

### (1) Oestrus inhibition

Thirty-two 6-week-old female mice were selected and divided into 4 groups, with 8 mice in each group. The female mice in the negative control group were subcutaneously injected with normal saline, and the female mice in the three Degarelix groups were subcutaneously injected with different dosages (0.4 mg/kg, 0.6 mg/kg and 1.0 mg/kg) of Degarelix once. After administration, vaginal smear experiments were performed on female mice every day to detect the oestrous condition. The results of vaginal smears showed that the female mice treated with Degarelix were in the diestrus for a certain period of time, and when the time elapsed, the female mice returned to the normal oestrus cycle.

The female mice in different dosage groups maintained the various duration of diestrus. The higher the dosage, the longer the diestrus lasted. It indicated that a single injection of Degarelix can effectively inhibit oestrus of female mice, and the inhibition time was positively correlated with the dosage (Table 2).

**Table 2**

| Groups | Treatment methods | Time of inhibiting oestrus (days) |
|---|---|---|
| Control group | Injection of normal saline | 4.63 ± 0.50^{a} |
| Low dose group | 0.4mg/kg, only subcutaneous injection once | 7.13 ± 2.09^{b} |
| Middle dose group | 0.6mg/kg, only subcutaneous injection once | 8.50 ± 3.54^{bc} |
| High dose group | 1.0mg/kg, only subcutaneous injection once | 18.50 ± 2.58^{d} |

| | | |
|---|---|---|
| Note: the different letters of the shoulder markers indicate significant differences (*P* < 0.05), and the same letters indicate that there is not significant difference (*P* > 0.05). | | |

### (2) Oestrus synchronization

It was discovered from the oestrus inhibition experiments that a dose of 1.0 mg/kg can inhibit oestrus of female mice for at least 15 days. Six 6-week-old female mice were selected for further oestrus synchronization experiments. The specific method was as follows: the female mice were subcutaneously injected 1.0 mg/kg of Degarelix, and intraperitoneally injected 5 units of PMSG on the 14th day after administration, and intraperitoneally injected 5 units of HCG after 46 hours, and then the oestrus of the female mice was detected.

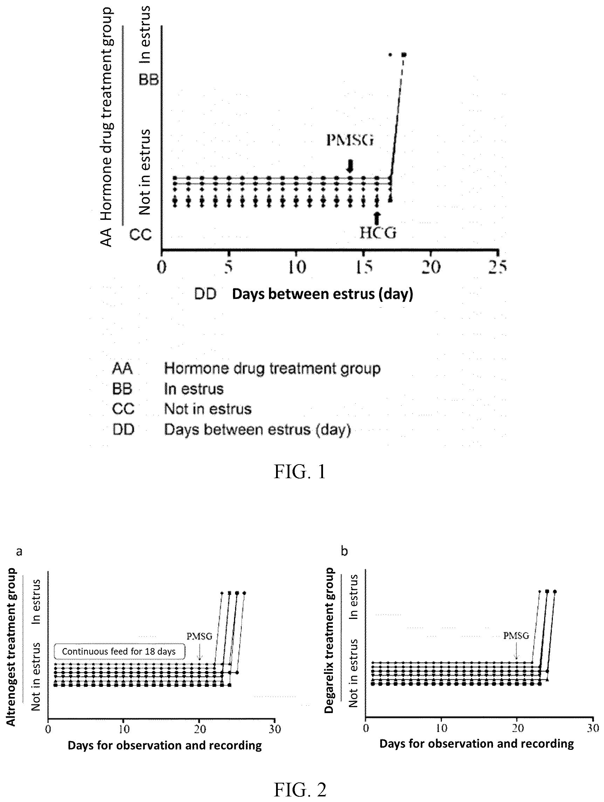

As shown in FIG. 1, the results showed that the female mice were concentrated in oestrus on the 17th and 18th days, which indicated that after administering of Degarelix to inhibit oestrus of female mice, the treatment with exogenous hormones can achieve oestrus synchronization of female mice.

### Example 3

This example served to illustrate use of Degarelix for inhibiting oestrus of sowpigs and for achieving ostrus synchronization.

The duration of oestrus inhibition in sowpigs after administration with different dosages of Degarelix was tested, and the oestrus-inhibited sowpigs were subjected to the treatment of estrus synchronization.

### (1) Oestrus inhibition

Thirty healthy adult sowpigs were selected and divided into a control group and Degarelix experimental groups, with 6 sowpigs in each group. The four Degarelix experimental groups were intramuscularly injected with different doses of Degarelix, namely 10 µg/kg body weight, 20 µg/kg body weight, 40 µg/kg body weight and 160 µg/kg body weight, respectively. The control group was intramuscularly injected with normal saline, and the oestrus performance (e.g., vulva performance and static reaction) of the sowpigs was observed and recorded to determine whether the sowpigs were in oestrus, and the time to restore oestrus after administration was recorded.

It was found that sowpigs in the Degarelix experimental groups came into oestrus significantly later than those in the control group after administration, and the higher the dose injected, the longer the time needed to restore oestrus.. The oestrus inhibition time of the sowpigs in 160µg/kg dose group was more than 90 days, which indicated that Degarelix can effectively inhibit oestrus in sowpigs, and the inhibition time was positively correlated with the dose (Table 3).

**Table 3**

| Groups | Treatment | Time to restore oestrus after administration (days) |
|---|---|---|
| Control group | Injection of normal saline | 14.33 ± 1.36^{a} |
| 10µg/kg Degarelix group | Intramuscular injection once | 21.50 ± 2.45^{b} |
| 20µg/kg Degarelix group | Intramuscular injection once | 38.83 ± 2.37^{c} |
| 40µₗg/kg Degarelix group | Intramuscular injection once | 53.83 ± 3.16^{d} |
| 160µg/kg Degarelix group | Intramuscular injection once | > 90 |

| | | |
|---|---|---|
| Note: the different letters of the shoulder markers indicate significant differences (P < 0.05). | | |

### (2) Oestrus synchronization

Comparison of experiments of administering Degarelix and altrenogest in achieving oestrus synchronization in gilts was performed. Twelve healthy adult gilts were selected and divided into two groups, namely an altrenogest group and a Degarelix group, with 6 gilts in each group. Among them, gilts in the altrenogest group were fed 20 mg of altrenogest every day for 18 consecutive days, while gilts in the Degarelix group were intramuscularly injected with 15 µg/kg body weight of Degarelix only on the first day. Both groups of gilts were intramuscularly injected with 1,000 units of PMSG on the 20th day after the first administration, then test whether the gilts in different treatment groups can achieve the estrus synchronization.

FIG. 2 illustrated the results of oestrus synchronization of gilts treated with PMSG following the administration of Degarelix and altrenogest to inhibit oestrus. The experimental results indicated that all the gilts in the altrenogest treatment group and the Degarelix treatment group were in oestrus within one week after administration of exogenous hormones, wherein gilts in the altrenogest treatment group were concentrated in oestrus for 4 days intensively from the 3rd day to the 6th day after injection with PMSG, and gilts in the Degarelix treatment group were concentrated in oestrus for 3 days intensively from the 3rd to the 5th day after injection with PMSG (FIG. 2), which demonstrated that after oestrus of the gilts was inhibited by Degarelix and altrenogest, use of an exogenous hormone PMSG can effectively promote synchronous oestrus of the gilts, and Degarelix can achieve synchronous oestrus of the gilts by only one intramuscular injection, and the concentration degree of oestrus was improved to a certain extent.

### Example 4

This example served to illustrate use of Degarelix for inhibiting oestrus of dogs.

### (1) Inhibiting effect of Degarelix on male dogs

Adult healthy male dogs with normal reproductive capacity were selected and divided into an experimental group and a control group. Each group of the animals was treated with subcutaneous injections, male dogs in the control group were injected with normal saline, and male dogs in the experimental group were injected with 1.5 mg/kg body weight of Degarelix, the treated animals in each group were fed and observed in the same way. Blood was collected from the animals regularly to measure and record the concentration of testosterone in serum (blood was collected and detected before the injection of drugs, and 1h, 2h, 4h, 6h, 8h, 24h, 48h, 72h, 120h and 168h after the injection of drugs, and after that, the blood was collected every 168h (1 week) until the end of the experiment), and scrotum circumference of male animals was measured and recorded regularly, sexual behavior of the male animals was observed and recorded, and compared with that of male animals in the control group.

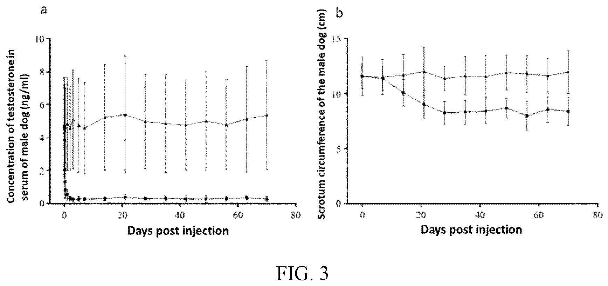

It was found that compared to the control group, testosterone concentration in Degarelix-treated male dogs decreased significantly, scrotal circumference was significantly reduced after a period of treatment (as shown in Fig. 3, where ▲ indicated the control group and ■ indicated the experimental group), and sexual behaviour was significantly reduced. Before the treatment on the experimental group, the male dogs had normal erections, ejaculation behavior, and semen could be collected. However, after the treatment with Degarelix, the male dogs in the experimental group had abnormal erections and no semen could be collected. The male dogs in control group had normal erections, ejaculation behavior, and semen could be collected before and after the administration of normal saline. Above results demonstrated that Degarelix can significantly inhibit oestrus of male dogs.

### (2) Inhibiting effect of Degarelix on female dogs

Healthy female dogs in proestrus (the proestrus period was less than 3 days) were selected and divided into an experimental group and a control group. Each group of the animals was treated with subcutaneous injections, the female dogs in control group were injected with normal saline, and the female dogs in experimental group were injected with 1.5 mg/kg body weight of Degarelix, each group of the treated animals was fed and observed in the same way to detect the oestrus of the female dogs.

The results showed that the oestrus symptoms of the female dogs treated with Degarelix gradually disappeared 3 days after the injection, including the gradual attenuation of vulvar redness and swelling, and the gradual reduction of vaginal discharge of mucus. The vaginal smear results indicated that the female dogs in experimental group quickly changed from proestrus to diestrus. The female dogs in control group had normal oestrus, with redness and swelling of the vulva and vaginal discharge of mucus. It demonstrated that the treatment with Degarelix in the proestrus stage can effectively inhibit oestrus of female dogs.

The above content describes in detail the preferred embodiments of the present disclosure, but the present disclosure is not limited thereto. A variety of simple modifications can be made in regard to the technical solutions of the present disclosure within the scope of the technical concept of the present disclosure, including a combination of individual technical features in any other suitable manner, such simple modifications and combinations thereof shall also be regarded as the content disclosed by the present disclosure, each of them falls into the protection scope of the present disclosure.

## Claims

1. Use of a GnRH antagonist in regulation and control of oestrus of a mammal.

2. The use according to claim 1, wherein the use is to inhibit oestrus or regulate and control oestrus synchronization.

3. The use according to claim 1 or 2, wherein the GnRH antagonist is at least one selected from the group consisting of Cetrorelix, Abarelix and Degarelix; preferably Degarelix.

4. The use according to any one of claims 1-3, wherein the mammal is a cat, a dog, a mouse, a rabbit, a horse, a cattle, a sheep or a pig.

5. The use according to any one of claims 1-4, wherein the GnRH antagonist is used in an amount of 1-5,000 µg per kg of animal body weight.

6. A pharmaceutical composition, wherein the pharmaceutical composition comprises a GnRH antagonist and a gonadotrophin; preferably, the GnRH antagonist is at least one selected from the group consisting of Cetrorelix, Abarelix and Degarelix; more preferably Degarelix;
preferably, the gonadotrophin is at least one selected from the group consisting of follicle stimulating hormone (FSH) and analogs thereof, pregnant mare serum gonadotropin (PMSG) and analogs thereof, and human chorionic gonadotropin (HCG) and analogs thereof;
preferably, the GnRH antagonist is used in an amount of 1-100 µg/kg body weight, and the gonadotrophin is used in an amount of 5-6,000 units.

7. Use of GnRH antagonist and gonadotrophin for regulation and control of oestrus synchronization of a mammal.

8. A method for inhibiting oestrus of a mammal, wherein the method comprises administering an effective amount of a GnRH antagonist to the non-estrous mammal to inhibit oestrus of the mammal;
preferably, the mammal is a cat or a dog.

9. A method for regulation and control of oestrus synchronization of mammals, wherein the method comprises: administering an effective amount of a GnRH antagonist to the non-estrous mammals to inhibit oestrus of mammals, and then administering an effective amount of gonadotrophin to achieve oestrus synchronization;
preferably, the mammal is a horse, a cattle, a sheep or a pig.

10. The method according to claim 9, wherein the method for administering gonadotrophin is as follows:
administering an effective amount of gonadotrophin to a mammal all at once; or initially administering an effective amount of at least one selected from the group consisting of FSH and analogs thereof and PMSG and analogs thereof to the mammal, and then administering HCG or analogs thereof after 24-96h.
